(19) Europäisches Patentamt
European Patent Office
Office européen des brevets

(11) Publication number: **0 295 092 B1**

(12) # EUROPEAN PATENT SPECIFICATION

(45) Date of publication of patent specification: **23.09.92**

(51) Int. Cl.5: **A61K 7/06**, A61K 7/48

(21) Application number: **88305255.7**

(22) Date of filing: **09.06.88**

(54) **Skin treatment composition.**

(30) Priority: **12.06.87 GB 8713747**

(43) Date of publication of application:
**14.12.88 Bulletin 88/50**

(45) Publication of the grant of the patent:
**23.09.92 Bulletin 92/39**

(84) Designated Contracting States:
**AT BE CH DE ES FR GB GR IT LI NL SE**

(56) References cited:
**EP-A- 0 182 756**
**FR-A- 2 094 109**
**FR-A- 2 591 889**
**US-A- 4 767 463**

**PATENT ABSTRACTS OF JAPAN vol. 12, no. 186 (C-500)(3033) 31 May 1988**

**SCIENCE vol. 228, 1985, WASHINGTON,DC,USA pages 1324 - 1326; D.C.WEST ET AL.: "Angiogenesis Induced by Degradation Products of Hyaluronic acid"**

(73) Proprietor: **UNILEVER PLC**
**Unilever House Blackfriars P.O. Box 68**
**London EC4P 4BO(GB)**
(84) Designated Contracting States:
**GB**

(73) Proprietor: **UNILEVER N.V.**
**Burgemeester s'Jacobplein 1 P.O. Box 760**
**NL-3000 DK Rotterdam(NL)**
(84) Designated Contracting States:
**BE CH DE ES FR GR IT LI NL SE AT**

(72) Inventor: **Scott, Ian Richard**
**6 Hoylake**
**Wellingborough Northants NN8 3NZ(GB)**

(74) Representative: **Tonge, Robert James et al**
**UNILEVER PLC Patent Division Colworth House Sharnbrook**
**GB-Bedford MK44 1LO(GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services

## Description

### FIELD OF INVENTION

The invention relates to cosmetic and pharmaceutical compositions for administration to mammalian skin or hair, the compositions containing special polysaccharides or derivatives thereof. The compositions are particularly useful for enhancing the quality and appearance of human skin following topical application, and are also useful in promoting or enhancing the growth of hair, more particularly on the human scalp.

### PRIOR ART AND BACKGROUND

It has been reported by West et al in Science, Volume 228 (1985), pages 1324-1326 that the partial degradation products of sodium hyaluronate produced by the action of testicular hyaluronidase induced an angiogenic response on the chick chorioallantoic membrane, this activity being restricted to hyaluronate fragments between 4 and 25 disaccharides in length.

GB-A-2 099 826 (Balazs) discloses an aqueous viscoelastic composition comprising a mixture of low (1000 to 200,000) and high (1,000,000 to 4,500,000) molecular weight fractions of sodium hyaluronate, together with protein from which the sodium hyaluronate is derived, as a skin care cosmetic which has emollient, moisturising, lubricating and elasticising effects on skin.

EP-A-0 237 644 (Angio-Medical Corporation) discloses a composition for skin care comprising omental lipids, and optionally other materials including a hygroscopic agent, such as hyaluronic acid, having a molecular weight of from 1000 to 1,000,000.

EP-A-0 197 718 (Fidia Spa) discloses topical compositions comprising a topically active pharmaceutical and hyaluronic acid or one of its molecular fractions, notably of molecular weight 250,000 to 350,000 or 50,000 to 100,000 or 500,000 to 730,000. These compositions are said to be useful in treating, inter alia, dermatological disorders.

US-4 605 691 (Biomatrix Inc) discloses the use of gels comprising cross-linked hyaluronic acid or its sodium salt of molecular weight 50,000 to 8,000,000 in drug delivery systems.

From a review of the foregoing references it is apparent that fragments of molecular weight as low as 1,000 to as high 8,000,000 have been proposed for a wide variety of uses, but none suggests any specific molecular weight fragment of hyaluronic acid that can rejuvenate aged, wrinkled skin or promote hair growth.

With the aim of improving the appearance of human skin by promoting the development of blood capillaries near the skin surface (angiogenesis), and of improving hair growth, particularly on the balding human scalp, following topical application of fragments of hyaluronic acid, it was discovered that penetration through the epidermal layers of the skin was particularly difficult. In contrast to the work of West et al using the chick chorioallantoic membrane, it was clear that human skin penetration presented an entirely new problem.

Although topical application of hyaluronic acid fragments has been proposed in the literature referred to above, there is no evidence of a significant angiogenic response via this route, nor of the promotion of hair growth or regrowth following topical application to the human scalp.

In contrast to the teaching of Balazs, who advocates a mixture of both low and high molecular weight fractions of sodium hyaluronate, and the Angio-Medical Corporation who suggest the optional use of hyaluronate having a wide range of molecular weights in topical products, it has now been discovered that by topical application of the carefully selected narrow molecular weight range of hyaluronic acid fragments, preferably together with an activity enhancer, compositions can be prepared which effectively penetrate the epidermal layer of the skin and surprisingly improve the appearance of the skin, particularly rejuvenation of aged, wrinkled skin, and an improvement in skin colour by an angiogenic response, to an extent that is quite unexpected. Evidence to support this benefit in terms of a local increase in the development of blood vessels in the skin following topical application of fragments of hyaluronic acid will be given later in this specification. Also, topical application of these fragments particularly to the human scalp in the region of vellus hair, can convert vellus hair to growth as terminal hair, or increase the rate of terminal hair growth to an extent which is also quite unexpected.

### DEFINITION OF THE INVENTION

The invention accordingly provides a composition for topical application to mammalian skin which comprises:

(i) from 0.01 to 99% by weight of hyaluronic acid fragments comprising from 7 to 50 monosaccharide units terminating either with a glucuronic acid unit and/or a N-acetyl glucosamine unit, or an unsaturated derivative of one or both of these terminal units; and

ii) from 1 to 99.99% by weight of a cosmetically acceptable vehicle;

provide that when the fragments of hyaluronic acid consist essentially of fragments composed of more than 25 monosaccharide units, then the composition also comprises a means for enhancing the activity of said fragments, in terms of angiogenic and/or hair growth response, following topical application of the composition to the skin

## DISCLOSURE OF THE INVENTION

### The fragments of hyaluronic acid

The composition according to the invention comprises fragments of the glycosaminoglycan derivative hyaluronic acid.

Hyaluronic acid itself consists of repeating units of glucuronic acid and N-acetyl glucosamine, having the structure (1):

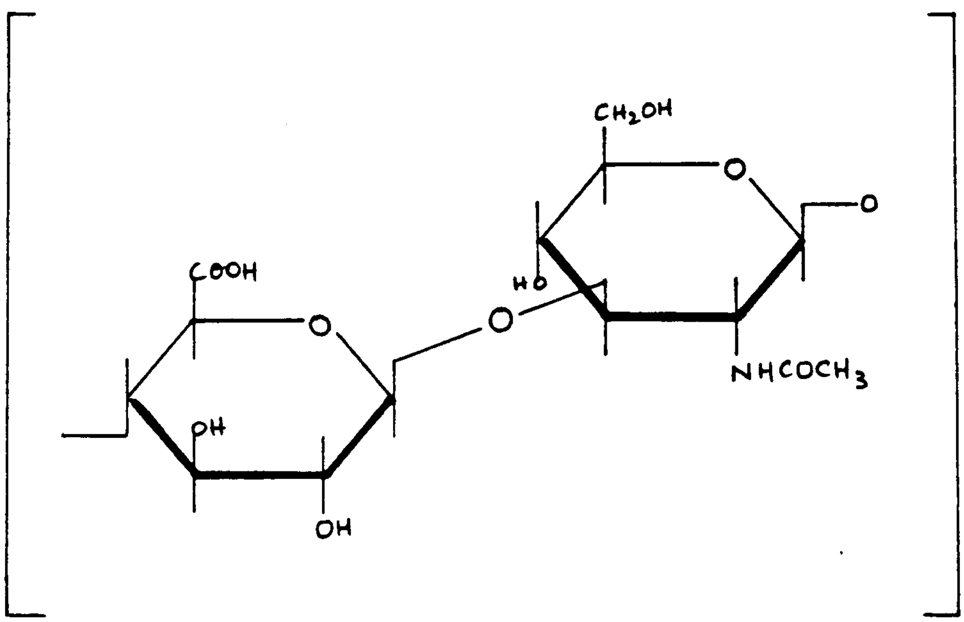

(1)

The fragments of hyaluronic acid are characterised as polysaccharides containing from 7 to 50 monosaccharides terminating either with a glucuronic acid unit and/or an N-acetyl glucosamine unit, or an unsaturated derivative of one or both of these terminal units.

It is apparent the the larger the fragments of hyaluronic acid, the greater the difficulty there is in delivering the fragments to the dermal layer of the skin, unless there is also present in the composition a means for enhancing the activity of said fragments. Accordingly, the preferred fragments of hyaluronic acid are polysaccharides containing from 7 to 25 monosaccharide units.

These fragments can be obtained by digestion of hyaluronic acid with the enzyme hyaluronidase, or by chemical cleavage of hyaluronic acid or by chemical synthesis from monosaccharides, disaccharides or short chain polysaccharides. The amount of of hyaluronic acid fragments to be incorporated in the composition according to the invention can be determined either by an angiogenic response, or by a hair growth response. Accordingly, when the fragments are to be employed in the area of skin benefit, the amount of the said fragments of hyaluronic acid present in the composition will be at least sufficient, after a period of at least 5 days, to increase the development of blood vessels in the skin of the rat the animal model selected for this test, when said composition is applied topically to the skin, by at least 5% more than that obtainable using a control composition from which the said fragments have been omitted.

Preferably, the amount of said fragments should be sufficient to increase the development of blood vessels in the skin of the rat by this technique by at least 10%, more preferably by at least 25%, most

preferably by at least 40% and ideally by at least 50%.

Alternatively, when the fragments of hyaluronic acid are to be employed in stimulating hair growth or regrowth, the amount of said fragments present in the composition according to the invention will be at least sufficient, after a period of at least 14 days, to increase hair growth in the rat, the animal model selected for this test, when said composition is applied topically to the skin, by at least 10% more than that obtainable using a control composition from which the said fragments have been omitted.

Preferably, the amount of said fragments of hyaluronic acid should be sufficient to increase hair growth in the rat by at least 20%, more preferably by at least 30%, most preferably by at least 40% and ideally by at least 50%.

The sufficient amount will depend on the effectiveness of the fragments some being more effective than others, but in general, an amount of from 0.01 to 99%, preferably from 0.1 to 20% by weight of the composition will provide an adequate dose to mammalian, particularly human skin or hair following topical application.

## The Vehicle

The composition according to the invention also comprises a solid, semi-solid or liquid cosmetically and/or physiologically acceptable vehicle, to enable the fragments of hyaluronic acid to be conveyed to the skin or hair at an appropriate dilution. The nature of the vehicle will depend upon the method chosen for topical application of the composition to the skin. The vehicle can itself be inert or it can possess physiological or pharmaceutical benefits of its own.

It should be explained that vehicles are substances which can act as diluents, dispersants, or solvents for the fragments of hyaluronic acid which therefore ensure that it they can be applied to and distributed evenly over the hair and/or scalp at an appropriate concentration. The vehicle is preferably one which can aid penetration of the fragments of hyaluronic acid into the skin to reach the dermal layer of the skin. Compositions according to the invention can include water as a vehicle, and/or at least one cosmetically acceptable vehicle other than water.

Vehicles other than water that can be used in compositions according to the invention can include liquids or solids as emollients, solvents, humectants, thickeners and powders. Examples of each of these types of vehicles, which can be used singly or as mixtures of one or more vehicles, are as follows:

Emollients, such as stearyl alcohol, glyceryl monoricinoleate, glyceryl monostearate, propane-1,2-diol, butane-1,3-diol, mink oil, cetyl alcohol, ispropyl isostearate, stearic acid, isobutyl palmitate, isocetyl stearate, oleyl alcohol, isopropyl laurate, hexyl laurate,

Solvents, such as ethyl alcohol, methylene chloride, isopropanol, castor oil, ethylene glycol monoethyl ether, diethylene glycol monobutyl ether, diethylene glycol monoethyl ether, dimethyl sulphoxide, dimethyl formamide, tetrahydrofuran;

Humectants, such as glycerin, sorbitol, sodium 2-pyrrolidone-5-carboxylate, soluble collagen, dibutyl phthalate, gelatin;

Powders, such as chalk, talc, fullers earth, kaolin, starch, gums, colloidal silicon dioxide, sodium polyacrylate, tetra alkyl and/or trialkyl aryl ammonium smectites, chemically modified magnesium aluminium silicate, organically modified montmorillonite clay, hydrated aluminium silicate, fumed silica, carboxyvinyl polymer, sodium carboxymethyl cellulose, ethylene glycol monostearate.

The amount of vehicle in the composition, including water if present, should preferably be sufficient to carry at least a portion of the fragments of hyaluronic acid to the skin in an amount which is sufficient effectively to enhance skin quality or hair growth. The amount of the vehicle can comprise the balance of the composition, particularly where little or no other ingredients are present in the composition. Accordingly, the vehicle or vehicles can comprise from 1 to 99.99%, preferably from 50 to 99.5% and ideally from 90 to 99% by weight of the composition.

## Activity Enhancer

The composition according to the invention also preferably comprises a means for enhancing the activity of the fragments of hyaluronic acid, especially to improve their penetration through the skin following topical application, with the consequence that skin benefit can be further improved and where appropriate hair growth enhanced.

It is accordingly apparent that the larger fragments of hyaluronic acid, that is those comprising more than 25 monosaccharide units, are too large to penetrate the skin to any significant extent unless there is also present an activity enhancer. Smaller molecular fragments of hyaluronic acid that is those comprising

from 7 to 25 monosaccharide units penetrate the skin more readily, but nonetheless their penetration can also be substantially enhanced in the presence of an activity enhancer.

The activity enhancer can be chosen from a wide variety of molecules which can function in different ways to enhance the benefits of the fragments of hyaluronic acid. Particular classes of activity enhancers include hair growth stimulants other than the said fragments, penetration enhancers and cationic polymers, whose presence can further improve the delivery of the fragments through the stratum corneum to their site of action.

Some activity enhancers can also function as vehicles for the fragments of hyaluronic acid.

The means for enhancing the activity of the fragments of hyaluronic acid can also take the form of an iontophoretic device as will be explained later. This and other means for enhancing the activity of the said fragments are now disclosed in greater detail.

(a) Other Hair Growth Stimulants

Examples of substances other than the fragments of hyaluronic acid substances which as activity enhancers themselves possess the ability to stimulate or increase hair growth include, for example;
Benzalkonium chloride
Benzethonium chloride
Phenol
Estradiol
Diphenhydramine hydrochloride
Chlorpheniramine maleate
Chlorophyllin derivatives
Cholesterol
Salicylic acid
Cystine
Red pepper tincture
Benzyl nicotinate
dl-Menthol
Peppermint oil
Calcium pantothenate
Panthenol
Castor oil
Hinokitiol
Prednisolone
Resorcinol

Further substances which themselves possess the ability to increase the rate of terminal hair growth include:

(i) α-1,4 esterified disaccharides described by Choay S.A. in EP-A-0 064 012, having the structure (2):

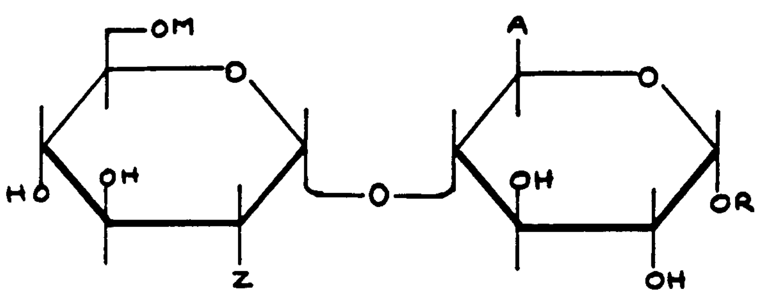

(2)

where

Z represents a functional nitrogen group, such as an azide or a group having the structure -NHB, in which B represents -H or a functional group such as acetyl or sulphate as a salt with an organic or mineral cation;

M represents -H or $SO_3M_1$, where $M_1$ is an organic or metallic cation, particularly an alkali metal; or an acetyl group;

R represents a $C_1$ to $C_4$ alkyl radical, especially methyl; or an aryl radical;

A represents a functional group such as an acid or -$COOR_1$, where $R_1$ represents -H or a $C_1$ to $C_4$ alkyl radical, especially methyl; or a metal, especially an alkali metal;

(ii) esterified oligosaccharides as described by Unilever in EP-A-0 211 610, including at least one esterified disaccharide unit consisting of a uronic acid residue having the structure (3):

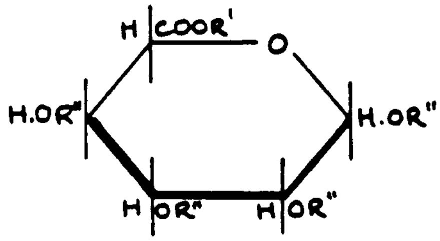

(3)

and a hexosamine residue having the structure (4):

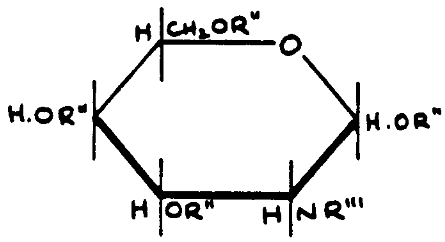

(4)

where

R′ is -H, C3 to $C_{10}$ alkyl or

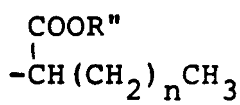

R″ is -H, $C_1$ to $C_4$ alkyl, -$CO(CH_2)_mCH_3$, or -$SO_3M$,

R‴ is -H, -$CO(CH_2)_mCH_3$, or -$SO_3M$,

M is -H, or a metallic or organic cation

n is 0 or an integer of from 1 to 7, and

m is 0 or the integer 1 or 2;

the groups designated R″ being the same or different, one R″ group from each pyranose ring structure being linked by a glycosidic linkage having the configuration $\alpha$-1,3, $\alpha$-1,4, $\beta$-1,3 or $\beta$-1,4; and the -COOR′, -$CH_2OR''$

and -OR″ groups being of either configuration with respect to the pyranose rings;

(iii) Minoxidil and its derivatives, as described by The Upjohn Co in GB 1 167 735,

(iv) Minoxidil glucuronides, as described by Unilever in EP-0 242 967,

(v) Minoxidil sulphates, as described by The Upjohn Co. in WO 86/04231.

(vi) Direct proteoglycanase inhibitors, such as 1,10-phenanthroline.

(vii)Glycosaminoglycanase inhibitors, such as aldonolactones and esterified aldonolactones having the structure (5):

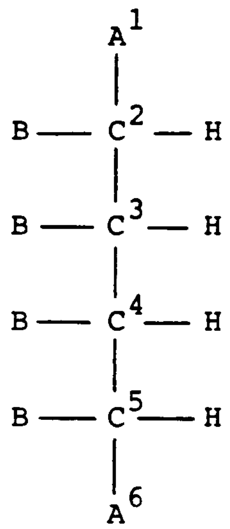

(5)

where

$A^1$ and $A^6$ are -H, $-CH_3$,

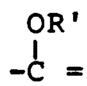 or 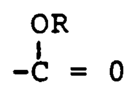

B is OR″ or a lactone linkage to position 1 or 6, or $-NHCOCH_3$

and where

R is -H or $C_2$ to $C_8$ alkyl,

R′ is the remainder of the molecule joined through another C atom at positions 2 to 5 to form a lactone, and

R″ is -H or $C_2$ (ie acetyl) to $C_4$ acyl of either configuration with respect to the backbone of this molecule; preferred examples of which include:

L-Galactono-1,4-lactone

L-Arabino-1,5-lactone

D-Fucono-1,5-lactone

D-Glucaro-1,4-lactone

D-Glucurono-6,3-lactone

Galactaric acid lactone

2-Acetamido-2-deoxygluconolactone

2-Acetamido-2-deoxygalactono-lactone

D-Glucaro-1,4:6,3-dilactone

L-Idaro-1,4-lactone

2,3,5-Tri-0-acetyl-D-glucaro-1,4-lactone

2,5-Di-0-acetyl-D-glucaro-1,4:6,3-dilactone

(viii)Glycosaminoglycanase inhibitors, such as monosaccharides and esterified monosaccharides having the structure (6):

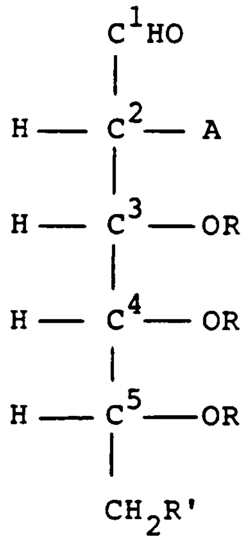

(6)

where
A is -OR or $-NHCOCH_3$
R is -H, $-SO_3M$, or $C_2$ (ie acetyl) to $C_4$ acyl
R′ is -H or -OR
M is -H or a metal cation
wherein the functional groups can be in either configuration with respect to the backbone of the above molecule;
preferred examples of which include:
N-Acetylglucosamine
N-Acetylgalactosamine
D-Galactosamine
D-Glucosamine-3-sulphate
N-Acetylmannosamine
(ix) glycosaminoglycan chain cellular uptake inhibitors such as, hexuronic acid and esters thereof which may be represented by the generic structure (7):

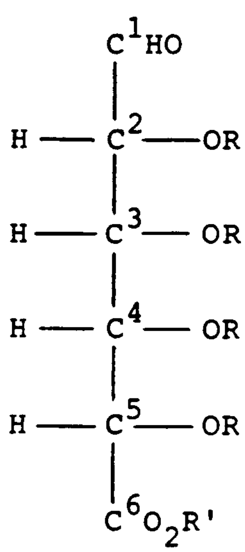

(7)

where
R is -H, $-SO_3M$, or $C_2$ (ie acetyl) to $C_4$ acyl;
R′ is -H or $C_2$ to $C_8$ alkyl.
wherein the functional groups can be in either configuration with respect to the backbone of the above

molecule;

(x) Chemical inhibitors of glycosidase activity chosen from lactams having the structure (8):

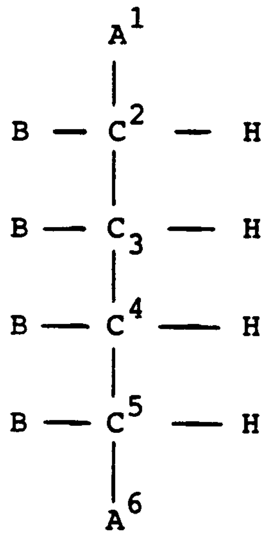

(8)

where $A^1$ and $A^6$ are -H, $-CH_3$,

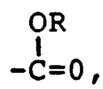

$-CH_2OR$ or

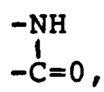

$A^1$ and $A^6$ being the same or different, and at least one of which being the group:

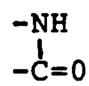

in a lactam ring;

and where B is -OR′, $-NHCOCH_3$ or a lactam linkage to $A^1$ or $A^6$;

the B groups being the same or different, and at least one of which is involved in a lactam linkage;

and where

R is -H, $-C_nH_{2n+1}$ or a metal ion,

R′ is -H or $-COC_nH_{2n+1}$, and

n is an integer of from 1 to 22;

provided that:

where any of the B groups is

-OR′ or $-NHCOCH_3$,

then that group or groups can be of either steriochemical configuration with respect to the plane of the ring,

preferred examples of which include:

D-glucaro-1,5-lactam

L-Galactono-1,4-lactam,

L-Arabino-1,5-lactam,
D-Fucono-1,5-lactam,
D-Glucaro-1,4-lactam,
D-Glucurono-6,3-lactam,
1,2,5-tri-O-acetyl-D-glucurono-6,3-lactam
2-Acetamido-2-deoxygluconolactam,
2-Acetamido-2-deoxygalactonolactam,
D-Glucaro-1,4:6,3-dilactam,
L-Idaro-1,4-lactam,
2,3,5-Tri-O-acetyl-D-glucaro-1,4-lactam,
2,5-Di-O-acetyl-D-Glucaro-1,4:6,3-dilactam,
D-glucaro-1,5-lactam ethyl ester,
(xi) chemical activators of protein kinase C enzymes chosen from diacylglycerols having the structure (9):

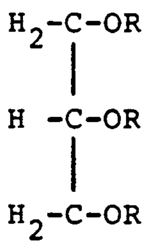

(9)

where
R is -H or

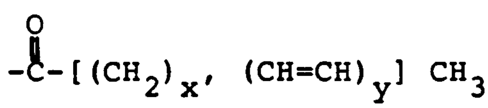

x is an integer of from 1 to 22, and
y is 0 or an integer of from 1 to 5;
provided that:
one of the R groups is -H and the remaining R groups are the same or different;
and provided also that the said remaining R groups can be of either steriochemical configuration with respect to the carbon backbone of the glycerol molecule;
preferred examples of which include:
1,2-Dihexanoyl-sn-glycerol
1,2-Dioctanoyl-rac-glycerol
1,2-Dioctanoyl-sn-glycerol
1-Oleoyl-2-acetyl-rac-glycerol
1-Oleoyl-2-acetyl-sn-glycerol
1-Stearoyl-2-arachidonoyl-sn-glycerol
1,2-Distearoyl-rac-glycerol
1,3-Dioctadecanoylglycerol 1,2-Dibutyrylglycerol
1,3-Dipentadecanoylglycerol
1,2-Dipalmitoyl-rac-glycerol
1,2-Dipalmitoyl-sn-glycerol
1,3-Dipalmitoylglycerol
1,2-Dioleoyl-sn-glycerol
1,2-Dioleoyl-rac-glycerol
1,3-Dioleoylglycerol
1,2-Diarachidonoylglycerol
1,2-Didecanoyl-rac-glycerol, and
1,3-Dieicosanoylglycerol.

(b) Penetration Enhancers

As has been stated earlier, the presence of a penetration enhancer, an example of an activity enhancer, can potentiate the benefit of the fragments of hyaluronic acid by improving their delivery to the dermal layer of the skin or when hair growth is involved, through the stratum corneum to its site of action in the immediate environment of the hair follicle close to the dermal papilla.

The penetration enhancer can accordingly function in a variety of ways. It can for example, improve the distribution of the fragments of hyaluronic acid on the skin surface or, it can increase their partition into the skin from the composition when applied topically, so aiding its passage to its site of action, or it can increase the permeability constant of the stratum corneum. Other mechanisms enhancing the benefit of these fragments may also be involved.

Examples of penetration enhancers include:
2-methyl propan-2-ol
Propan-2-ol
Ethyl-2-hydroxypropanoate
Hexan-2,5-diol
POE(2) ethyl ether
Di(2-hydroxypropyl) ether
Pentan-2,4-diol
Acetone
POE(2) methyl ether
2-hydroxypropionic acid
2-hydroxyoctanoic acid
Propan-1-ol
1,4 Dioxane
Tetrahydrofuran
Butan-1,4-diol
Propylene glycol dipelargonate
Polyoxypropylene 15 stearyl ether
Octyl alcohol
POE ester of oleyl alcohol
Oleyl alcohol
Lauryl alcohol
Dioctyl adipate
Dicapryl adipate
Diisopropyl adipate
Diisopropyl sebacate
Dibutyl sebacate
Diethyl sebacate
Dimethyl sebacate
Dioctyl sebacate
Dibutyl suberate
Dioctyl azelate
Debenzyl sebacate
Dibutyl phthalate
Dibutyl azelate
Ethyl myristate
Dimethyl azelate
Butyl myristate
Dibutyl succinate
Didecyl phthalate
Decyl oleate
Ethyl caproate
Ethyl salicylate
Isopropyl palmitate
Ethyl laurate
2-ethyl-hexyl pelargonate
Isopropyl isostearate

Butyl laurate
Benzyl benzoate
Butyl benzoate
Hexyl laurate
Ethyl caprate
Ethyl caprylate
Butyl stearate
Benzyl salicylate
2-hydroxypropanoic acid
2-hyroxyoctanoic acid,

Yet further penetration enhancers include esters of pyroglutamic acid having the structure (10):-

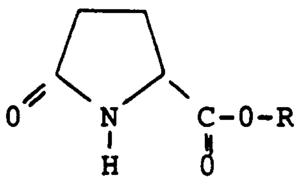

(10)

where
R is $C_1$ to $C_{30}$ alkyl.

Examples of suitable esters of pyroglutamic acid where are:
pyroglutamic acid methyl ester
pyroglutamic acid ethyl ester
pyroglutamic acid n-propyl ester
pyroglutamic acid n-butyl ester
pyroglutamic acid n-hexyl ester
pyroglutamic acid n-heptyl ester
pyroglutamic acid n-octyl ester
pyroglutamic acid n-nonyl ester
pyroglutamic acid n-decyl ester
pyroglutamic acid n-undecyl ester
pyroglutamic acid n-dodecyl ester
pyroglutamic acid n-tridecyl ester
pyroglutamic acid n-tetradecyl ester
pyroglutamic acid n-hexadecyl ester
pyroglutamic acid n-octadecyl ester
pyroglutamic acid n-eicosyl ester
pyroglutamic acid iso-propyl ester
pyroglutamic acid 2-methylhexyl ester
pyroglutamic acid 2-ethylhexyl ester
pyroglutamic acid 3,7-dimethyloctyl ester
pyroglutamic acid 2-hexyldecyl ester
pyroglutamic acid 2-octyldodecyl ester
pyroglutamic acid 2,4,4-trimetyl-1-pentane ester
pyroglutamic acid methyloctyl ester

Particularly preferred esters of this group are those where R in structure (10) is $C_1$ to $C_{14}$ alkyl, (linear or branched), especially $C_1$ to $C_6$ (linear or branched).

It is to be understood that the above lists of specific examples of esters of pyroglutamic acid are not exhaustive, there being many other examples expressed by the generic structure of these esters.

Further examples of penetration enhancers include:-
Dimethyl sulphoxide
N,N-Dimethyl acetamide
N,N-Dimethyl formamide
2-Pyrrolidone
1-Methyl-2-pyrrolidone
5-Methyl-2-pyrrolidone

1,5-Dimethyl-2-pyrrolidone
1-Ethyl-2-pyrrolidone
Phosphine oxides
Sugar esters
Tetrahydrofurfural alcohol
Urea
Diethyl-m-toluamide, and
1-Dodecylazacycloheptan-2-one

Further examples of penetration enhancers are surface active agents, preferred examples of which include:

(i) Anionic surface active agents, such as metallic or alkanolamine salts of fatty acids for example sodium laurate and triethanolamine oleate; alkyl benzene sulphonates, for example triethanolamine dodecyl benzene sulphonate;
alkyl sulphates, for example sodium lauryl sulphate;
alkyl ether sulphates, for example sodium lauryl ether sulphate [2 to 8 EO];
sulphosuccinates, for example sodium dioctyl sulphonsuccinate;
monoglyceride sulphates, for example sodium glyceryl monostearate monosulphate;
isethionates, for example sodium isethionate;
methyl taurides, for example Igepon T;
acylsarcosinates, for example sodium myristyl sarcosinate;
acyl peptides, for example Maypons and Lamepons;
acyl lactylates,
polyalkoxylated ether glycollates, for example trideceth-7 carboxylic acid;
phosphates, for example sodium dilauryl phosphate.
(ii) Cationic surface active agents, such as amine salts, for example sapamin hydrochloride;
quatenary ammonium salts, for example Quaternium 5, Quaternium 31 and Quaternium 18;
(iii) Amphoteric suface active agents, such as imidazol compounds, for example Miranol;
N-alkyl amino acids, such as sodium cocaminopropionate and asparagine derivatives;
betaines, for example cocoamidopropylbetaine
(iv) Nonionic surface active agents, such as fatty acid alkanolamides, for example oleic ethanolamide;
esters of polyalcohols, for example Span;
polyglycerol esters, for example that esterified with $C_{12-18}$ fatty acids and one or several OH groups;
polyalkoxylated derivatives, for example polyoxy:polyoxyethylene stearate, and octylphenoxy polyethoxyethanol (TRITON X-100);
ethers, for example polyoxyethylene lauryl ether;
ester ethers, for example Tween;
amine oxides, for example coconut and dodecyl dimethyl amine oxides.

Mixtures of two or more of the above surface active agents can be employed in the composition according to the invention.

(c) Cationic Polymers

Certain cationic polymers also function as activity enhancers. Particularly preferred cationic polymers for this purpose are chosen from:

Guar Hydroxypropyltrimonium chloride
Quaternium-19
Quaternium-23
Quaternium-40
Quaternium-57
Poly(dipropyldiallylammonium chloride)
Poly(methyl-$\beta$-propaniodiallylammonium chloride)
Poly(diallylpiperidinium chloride)
Poly(vinyl pyridinium chloride)
Quaternised poly (vinyl alcohol)
Quaternised poly (dimethylaminoethylmethacrylate); and mixtures thereof.

The amount of activity enhancer, when employed in accordance with the invention, will normally be from 0.1 to 50%, preferably from 0.5 to 25% and most preferably from 0.5 to 10% by weight of the composition.

(d) Iontophoresis

A further means for enhancing the activity of fragments of hyaluronic acid following topical application is the use of iontophoresis. A preferred iontophoretic device for this purpose comprises a pad of absorbent material, such as a nonwoven sheet or sponge, impregnated with a solution of fragments of hyaluronic acid, as herein defined, the pad carrying an electrode, for example in the form of a metallic sheet, through which an electric current can be passed, in order to enhance delivery of the fragments of hyaluronic acid to and through the epidermal layer of the skin.

Other adjuncts

The composition according to the invention can also contain adjuncts other than those already mentioned, depending on the form of the intended product. It is, for example, possible to include antiseptics, preservatives, antioxidants, emulsifiers, perfumes and colouring agents, which can improve the stability and/or consumer appeal of the composition.

The composition according to the invention can also be employed as a vehicle for a wide variety of cosmetically or pharmaceutically active ingredients.

PROCESS

The invention also provides a process for the preparation of a composition suitable for topical application to mammalian skin or hair which process comprises the steps of:

(i) preparing fragments of hyaluronic acid, said fragments being characterised as polysaccharides containing from 7 to 50 monosaccharide units terminating either with a glucuronic acid unit and/or a N-acetyl glucosamine unit, or an unsaturated derivative of one or both of said terminal units; and

(ii) combining said fragments with a cosmetically acceptable vehicle.

Convenient methods for preparing the fragments of hyaluronic acid include;

(a) digestion of hyaluronic acid with an hyaluronidase,

(b) chemical cleavage of hyaluronic acid, and

(c) chemical synthesis from monosaccharides, disaccharides or shorter chain polysaccharides.

The preferred methods of preparing the fragments of hyaluronic acid are by digestion and by chemical cleavage to be carried out as follows:

High molecular weight hyaluronic acid is mixed with the enzyme hyaluronidase, or with a solution of hot dilute acid or alkali. Either treatment results in digestion of the hyaluronic acid into a mixture of smaller fragments.

The digest is passed either batchwise or by continuous perfusion through an ultrafiltration membrane selected to pass fragments of the desired size. Where digestion is by extremes of pH, the ultrafiltrate is cooled and neutralised to prevent further reduction in size of the selected hyaluronic acid fragments. Where enzyme digestion is employed, the enzyme is removed by the ultrafiltration automatically so that further digestion of the selected fragments does not take place. Another advantage is that further batches of high molecular weight hyaluronic acid can then be digested by the same enzyme preparation in order to obtain further supplies of hyaluronic acid fragments.

Product Form and Container

The compositions of the invention can be formulated as liquids, for example as a lotion, shampoo, milk, cream, lotion, microemulsion, liposomal suspension or mousse for use in conjunction with an applicator such as a roll-ball applicator, or a spray device such as an aerosol can containing propellant, or a container fitted with a pump to dispense the liquid product.

Alternatively, the compositions of the invention can be solid or semi-solid, for example sticks, creams or gels, for use in conjunction with a suitable applicator or simply a tube, bottle or lidded jar, or as a liquid-impregnated fabric, such as a tissue wipe.

The invention accordingly also provides a closed container containing a composition as herein defined.

Use of the composition according to the invention

The composition according to the invention is particularly useful when applied topically to mammalian skin, particularly human skin, in order by a positive angiogenic response to induce improvements to the

skin, for example, rejuvenation of aged skin or reduction of wrinkles in wrinkled skin.

The composition according to the invention can also be applied to the scalp in the region of vellus hair so as to convert vellus hair to growth as terminal hair. Furthermore, the composition can also be applied to terminal hair, particularly on the scalp, in order to increase the rate of growth of that hair.

Topical application of the composition according to the invention can, as already explained, be accompanied by iontophoresis.

The amount of the composition and the frequency of application to the skin, hair and/or scalp can vary widely, depending on personal needs, but it is suggested as an example that topical application of from 0.1 to 5g daily containing from 0.1 to 1g of the fragments of hyaluronic acid over a period of at least six months will in most cases result in an improvement in hair growth and/or skin condition.

## EVIDENCE TO SUPPORT ANGIOGENIC ACTIVITY IN SKIN FOLLOWING ADMINISTRATION OF HYALURONIC ACID FRAGMENTS

One of the benefits of applying hyaluronic acid fragments topically to the skin is a local increase in the development of blood vessels, which can result in a warmer appearance to otherwise pale or palid skin. The development of skin wrinkles associated with the ageing process can also be arrested and in some instances reversed. This is seen as a rejuvenation benefit. Evidence to support these benefits was obtained as follows:

### 1. By topical application to the skin of the rat

#### Methodology

Hyaluronic acid fragments of size range 7 to 50 monosaccharide units were prepared by testicular hyaluronidase digestion followed by gel filtration. They were dissolved in a mixture of dimethylsulphoxide (75 parts by weight) and water (25 parts by weight) to provide a 5% by weight test solution of the fragments.

10µl of this solution was applied to 1cm$^2$ of shaved dorso-lateral rat skin with a control aliquot of the dimethylsulphoxide/water mixture, free from hyaluronic acid fragments, to a similar contralateral site of the same animal.

Similar amounts of test and control solution were applied twice daily for 5 days, after which treatments were discontinued for 3 days. The animals were then sacrificed, the treated skin removed and cryostat sections of 25µm thickness were stained for alkaline phosphatase activity after formal/calcium fixation using naphthol ASMX phosphate. Blood capillaries were counted in the superficial 0.2mm of the dermis. 5 rats in all were treated in this manner.

#### Results

Average field counts of blood capillaries on both test and control sites in each animal are set out in the table below:

| Rat | No. of blood capillaries $x10^{-5}/\mu m^2$ (average of 5 field counts) | |
|---|---|---|
| | Test Site | Control Site |
| 1 | 16.14 | 15.36 |
| 2 | 14.66 | 14.92 |
| 3 | 16.54 | 14.72 |
| 4 | 16.84 | 15.86 |
| 5 | 17.56 | 14.18 |
| Mean | 16.35 | 15.01 |

Conclusions

Statistical analysis of these data by paired t-test indicates that the number of capillaries on the test site was significantly greater than the number on the control site ($p = 0.045$), thus providing proof of the angiogenic property of topically applied hyaluronic acid fragments.

2. By subcutaneous implantation in the skin of the rabbit

Methodology

Hyaluronic acid fragments of size range 7 to 50 monsaccharide units were prepared by testicular hyaluronidase digestion followed by gel filtration. They were dissolved in a solution of methyl cellulose and dried into discs of approximately 2 mm diameter and 20 µm thickness. Discs containing 0, 10 or 100 µg of hyaluronic acid fragments were surgically inserted into the dorsal skin of rabbits and left for 5 days. At this time, the skin was removed and samples processed for microscopy either with haematoxylin and eosin staining or Masson-trichrome or with a monoclonal antibody to endothelial cells.

Results

Representative fields from each sample were examined blind and the number of blood vessels counted. Results are shown below:

| Dose of hyaluronic acid fragments | No. of blood vessels x$10^{-5}$/µm (average of 5 field counts) |
|---|---|
| 0 (Control) | 4.914 |
| 10 µg | 5.652 |
| 100 µg | 7.165 |

Conclusions

Statistical analysis of the data show that the 100 µg hyaluronic acid treated samples have a significantly greater number of blood vessels ($p = 0.028$ by t-test, $p = 0.056$ by Mann Whitney U-test).

It is apparent from these results that the topical application of hyaluronic acid fragments at the 100 µg level produces a significant increase in the number of blood vessels at or near the skin surface.

EXAMPLES

The invention is illustrated by the following examples. The abbreviation "HA" refers to "hyaluronic acid".

Example 1

This example illustrates the use of liposomes as a means for delivering fragments of hyaluronic acid to the skin surface.

A 5% by weight solution of hyaluronic acid (HA) fragments (7 to 50 monosaccharides) is sonicated with 10% by weight phophatidyl choline to produce liposomes. These are concentrated by ultrafiltration and added to the following formulation to form a skin lotion.

| | % w/w |
|---|---|
| Liposomes/HA fragments (7 to 20 monosaccharide units) | 2 |
| Carrageenan | 1 |
| Sodium chloride | 1 |
| Water | 96 |

Example 2

This examples illustrates the use of penetration enhances with large HA fragments.

| | % w/w |
|---|---|
| HA fragments (15 to 50 monosaccharide units) | 5 |
| Ethyl pyroglutamate | 20 |
| Ethanol | 25 |
| Triton X100 | 2 |
| Water | 48 |

Example 3

This examples illustrates the use of iontophoresis as a means for enhancing penetration of HA fragments through the dermal layer of the skin.

HA fragments (7 to 25 monosaccharides) are dissolved at 5% by weight level in water and impregnated into an absorbent paper pad bonded to a flexible aluminium sheet which is attached to the negative pole of a 6 volt battery, the other pole being earthed. The pad is placed in contact with the skin for periods of 6 to 18 hours for several days in order to induce blood vessel growth in the contact region. The application is particularly useful for the balding scalp to aid hair growth.

Examples 4 to 7

The following formulations represent anti-ageing creams, according to the invention.

| | % w/w | | | |
|---|---|---|---|---|
| | 4 | 5 | 6 | 7 |
| Cetyl alcohol polyoxyethylene (10) | 4 | 4 | 4 | 4 |
| Cetyl alcohol | 4 | 4 | 4 | 4 |
| Mineral oil | 4 | 2 | - | - |
| Paraffin wax | - | 2 | 4 | - |
| Partial glyceride of palmitic and stearic acids | - | - | - | 4 |
| 2-hydroxyoctanoic acid | 1 | 1 | 2 | 2 |
| HA fragments (7 to 50 monosaccharides units) | 10 | 15 | 5 | 2 |
| Triethanolamine | 0.75 | 0.75 | 0.75 | 0.75 |
| Butane-1,3-diol* | 3 | 3 | 3 | 3 |
| Xanthan gum | 0.3 | 0.3 | 0.3 | 0.3 |
| Preservative | 0.4 | 0.4 | 0.4 | 0.4 |
| Water to | 100 | 100 | 100 | 100 |
| pH adjusted with triethanolamine to | 4.0 | 4.0 | 4.0 | 4.0 |

* penetration enhancer

Example 8

This example illustrates a lotion according to the invention which is suitable for topical application to the scalp in order to promote hair growth. The lotion had the following formulation:

| | % w/w |
|---|---|
| Mixed fragments of hyaluronic acid containing from 7-50 monosaccharide units | 1 |
| Dibutyl sebacate | 5 |
| Ethanol | 45 |
| Perfume | qs |
| Water | 49 |

Example 9

This example illustrates an anti-wrinkle skin lotion.

The skin lotion had the following formulation:

| | % w/w |
|---|---|
| Fragments of hyaluronic acid (7 to 50 monosaccharide units) | 2 |
| Water | 49 |
| Sodium chloride | 2 |
| Perfume | qs |
| Ethanol | to 100 |

Example 10

This example illustrates a lotion according to the invention which is suitable for topical application to the scalp in order to promote hair growth.

The lotion has the following formulation:

| | % w/w |
|---|---|
| HA fragments (7 to 25 monosaccharides units) | 0.1 |
| 2-hydroxyoctanoic acid | 2 |
| ethanol | 30 |
| perfume | q.s. |
| water | to 100 |

Example 11

This Example illustrates a hair tonic which is suitable for application to hair or scalp.

The hair tonic has the following formulation:

| | % w/w |
|---|---|
| HA fragments (7 to 25 monosaccharide units) | 0.8 |
| ethanol | 50 |
| water | 49 |
| perfume | q.s. |

Example 12

This Example also illustrates a lotion which is suitable for topical application to the scalp.

The lotion had the following formulation:

| | % w/w |
|---|---|
| HA fragments (7 to 50 monosaccharide units) | 1.5 |
| minoxidil | 1 |
| propan-2-ol | 10 |
| ethanol | 88.5 |
| perfume | q.s. |

Example 13

This Example also illustrates a hair tonic which is suitable for application to hair or scalp. The hair tonic had the following formulation:

| | % w/w |
|---|---|
| HA fragments (7 to 50 monosaccharide units) | 0.2 |
| glucaro-1,4-dilactone | 2 |
| ethanol | 40 |
| water | 59.80 |
| perfume | q.s. |

Example 14

The following formulation represents a lotion which can be used topically in the treatment of bald or balding male or female heads.

| | % w/w |
|---|---|
| Hydroxyethyl cellulose | 0.4 |
| Absolute ethanol | 25 |
| Propane-1,2-diol | - |
| Butane-1,3-diol | 38.4 |
| Paramethyl benzoate | 0.2 |
| HA fragments (26 to 50 monosaccharide units) | 2 |
| Minoxidil | 1 |
| Perfume | 1 |
| Water | to 100 |

Examples 15 to 18

The following formulations represent lotions which can be used topically in the treatment of bald or balding male or female heads.

| | % w/w | | | |
|---|---|---|---|---|
| | 15 | 16 | 17 | 18 |
| Hydroxyethyl cellulose | 0.4 | - | 0.4 | - |
| Absolute ethanol | 25 | 25 | 25 | 25 |
| Propane-1,2-diol | - | - | 38.4 | 38.4 |
| Butane-1,3-diol | 38.4 | 38.8 | - | - |
| Paramethyl benzoate | 0.2 | 0.2 | 0.2 | 0.2 |
| HA fragments (7 to 50 monosaccharide units) | 25 | 10 | 8 | 1 |
| Perfume | 1 | 1 | 1 | 1 |
| Water | to 100 | 100 | 100 | 100 |

Example 19

This example illustrates a composition according to the invention in the form of a water-in-oil high internal phase emulsion.

The emulsion consisted of 10% by volume oily phase and 90% by weight aqueous phase.

The oily phase and the aqueous phase had the following consitution:

| | % w/w |
|---|---|
| Oily phase | |
| Sorbitan monooleate | 20 |
| Quaternium-18 hectorite | 5 |
| Liquid paraffin | 75 |
| | |
| Aqueous phase | |
| HA fragments (26 to 50 monosaccharide units) | 15 |
| Xanthan gum | 1 |
| Preservative | 0.3 |
| Perfume | q.s. |
| Sodium chloride (1% w/w solution) | to 100 |

The emulsion was prepared by taking 10 parts by volume of the oily phase and to it adding slowly with stirring 90 parts by volume of the aqueous phase.

The high internal phase water-in-oil emulsion so formed can be applied topically to the scalp, to improve hair growth and regrowth.

The following examples 20 to 22 illustrate shampoos for use in washing the hair and scalp, and for promoting hair growth on the scalp.

Example 20

| | % w/w |
|---|---|
| Sodium lauryl ether sulphate (2 EO) : 21% AD | 41.4 |
| Lauryl dimethylamino acetic acid betaine * 30% AD | 4 |
| Coconut fatty acid diethanolamine | 1.5 |
| Oleyl triethoxy phosphate (BRIPHOS 03D) | 1 |
| Polyglycol-polyamine condensation resin (POLYQUART H) : 50% active | 1.5 |
| Preservative, colouring matter, salt | 0.58 |
| HA fragments (7 to 30 monosaccharide units) | 5 |
| Perfume | q.s. |
| Water | to 100 |

Example 21

| | % w/w |
|---|---|
| Sodium lauryl ether sulphate (2 EO) : 100% AD | 12 |
| POLYQUART H : 50% active | 2.5 |
| BRIPHOS 03D | 2.5 |
| HA fragments (10 to 40 monosaccharide units) | 4 |
| Zinc Sulphate | 5 |
| Perfume | q.s. |
| Water | to 100 |

Example 22

| | % w/w |
|---|---|
| Monoethanolamine lauryl sulphate : 100% AD | 20 |
| POLYQUART H : 50% active | 3 |
| BRIPHOS 03D | 1.7 |
| Coconut diethanolamide | 5 |
| HA fragments (7 to 50 monosaccharide units) | 25 |
| Perfume | q.s. |
| Water | to 100 |
| pH adjusted to 6.5 | |

Examples 23 to 34

These examples illustrate compositions according to the invention in the form of lotions, each containing an activity enhancer, which can be used topically in the treatment of bald or balding male or female heads, in order to initiate or promote or enhance hair growth.

| | %w/w | | |
|---|---|---|---|
| Example No. | 23 | 24 | 25 |
| Minoxidil | 1 | 2 | 5 |
| Absolute ethanol | 10 | 20 | 30 |
| HA fragments (7 to 50 monosaccharide units) | 1 | 5 | 0.5 |
| Paramethyl benzoate | 0.2 | 0.2 | 0.2 |
| Perfume | q.s | q.s | q.s |
| Water | to 100 | to 100 | to 100 |

| Example No. | 26 | 27 | 28 |
|---|---|---|---|
| Esterified disaccharide(11) | 1 | 2 | 5 |
| Absolute ethanol | 10 | 15 | 20 |
| HA fragments (26 to 50 monosaccharide units) | 15 | 5 | 1 |
| Paramethyl benzoate | 0.2 | 0.2 | 0.2 |
| Perfume | q.s | q.s | q.s |
| Hydroxethyl cellulose | - | 0.4 | - |
| Water | to 100 | to 100 | to 100 |

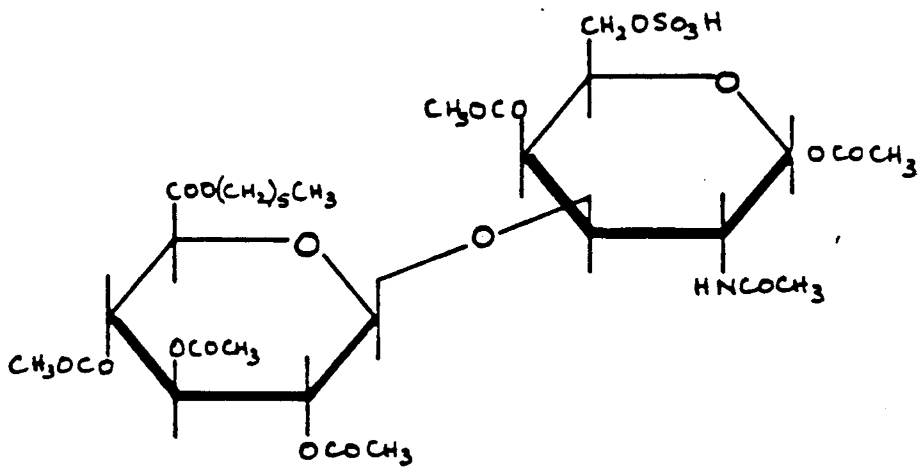

(11)

| Example No. | 29 | 30 | 31 |
|---|---|---|---|
| Zinc sulphate | 1 | 5 | 10 |
| Absolute ethanol | 5 | - | - |
| HA fragments (7 to 50 monosaccharide units) | 10 | 5 | 1 |
| Perfume | q.s | q.s | q.s |
| Paramethyl benzoate | - | 0.2 | 0.2 |
| Water | to 100 | to 100 | to 100 |

| Example No. | 32 | 33 | 34 |
|---|---|---|---|
| N-methyl pyrrolidone | 1 | 5 | 10 |
| Absolute ethanol | - | - | 5 |
| Hair growth promoter | 10 | 5 | 0.5 |
| Hydroxyethyl cellulose | 0.4 | 0.4 | 0.4 |
| Paramethyl benzoate | 0.2 | 0.2 | 0.2 |
| Perfume | q.s | q.s | q.s |
| Water | to 100 | to 100 | to 100 |

## Claims

**1.** A composition for topical application to mammalian skin which comprises:
(i) from 0.01 to 99% by weight of hyaluronic acid fragments comprising from 7 to 50 monosaccharide units terminating either with a glucuronic acid unit and/or an N-acetyl glucosamine unit, or an unsaturated derivative of one or both of these terminal units; and
(ii) from 1 to 99.99% by weight of a cosmetically acceptable vehicle;
provided that when the fragments of hyaluronic acid consist essentially of fragments composed of more than 25 monosaccharide units, then the composition also comprises a means for enhancing the activity of said fragments, in terms of angiogenic and/or hair growth response, following topical application of the composition to the skin.

**2.** A composition according to claim 1, in which the hyaluronic acid fragments comprise from 7 to 25 monosaccharide units.

**3.** A composition according to claim 2, in which the composition additionally comprises a means for enhancing the activity of said fragments following topical application to the skin, in terms of angiogenic and/or hair growth response.

**4.** A composition according to claim 1,2 or 3, which comprises from 0.1 to 20% by weight of hyaluronic acid fragments.

**5.** A compositions according any preceding claim, in which the vehicle forms from 80 to 99.9% by weight of the composition.

**6.** A composition according to any preceding claim, in which the means for enhancing the activity of the fragments of hyaluronic acid is a hair growth stimulant.

**7.** A composition according to claim 6, in which the hair growth stimulant is chosen from:
(i) $\alpha$-1,4 esterified disaccharides having the structure (2);
(ii) esterified oligosaccharides including at least one esterified disaccharide unit consisting of a uronic acid residue having the structure (3) and a hexosamine residue having the structure (4);
(iii)minoxidil and derivatives therof;
(iv) direct proteoglycanase inhibitors;
(v) glycosaminoglycanase inhibitors;
(vi) glycosaminoglycan chain cellular uptake inhibitors;
(vii)glycosidase inhibitors; and
(viii)chemical activators of protein kinase C enzymes.

**8.** A composition according to claim 7, in which the hair growth stimulant in minoxidil.

**9.** A composition according to claim 7, in which the glycosaminoglycanase inhibitor is an aldonolactone having the structure (5).

**10.** A composition according to claim 9, in which the aldonolactone is D-glucaro-1,4-lactone.

**11.** A composition according to claim 7, in which the glycosaminoglycanase inhibitor is a monosaccharide having the structure (6).

**12.** A composition according to claim 11, in which the monosaccharide is N-acetylglucosamine.

**13.** A composition according to claim 7, in which the glycosidase inhibitor is a lactam having the structure (8).

**14.** A composition according to claim 13, in which the lactam is D-glucaro-1,5-lactam.

**15.** A composition according to claim 7, in which the chemical activator of protein kinase C enzymes is a diacylglycerol having the structure (9).

**16.** A composition according to claim 15, in which the diacylglycerol is 1,2-dihexanoyl-sn-glycerol.

**17.** A composition according to any of claims 1 to 5, in which the means for enhancing the activity of the fragments of hyaluronic acid is a penetration enhancer.

**18.** A composition according to claim 17, in which the penetration enhancer is chosen from:
1-dodecylazacycloheptan-2-one
dibutyl sebacate
2-hydroxyoctanoic acid
esters of pyroglutamic acid having the structure (10)
surface active agents.

**19.** A composition according to any of claims 1 to 5, in which the means for enhancing the activity of the fragments of hyaluronic acid is a cationic polymer.

**20.** A composition according to any of claims 1 to 5 in which the means for enhancing the activity of the fragments of hyaluronic acid is an iontophoretic device.

**21.** A composition according to any preceding claim which when applied topically to the skin of the rat, the animal model selected for this test, is capable of inducing an angiogenic response, that is an increase in the development of blood vessels in the skin after at least 5 days, of at least 5% more than that obtainable using a control composition from which the fragments of hyaluronic acid have been omitted.

**22.** A composition according to claim 21, in which the composition is capable of increasing the development of blood vessels by at least 10%.

**23.** A composition according to any preceding claim which when applied topically to the skin of the rat, the animal model selected for this test, is capable of inducing a hair growth response, that is an increase in hair growth after at least 14 days, of at least 10% more than that obtainable using a control composition from which the fragments of hyaluronic acid have been omitted.

**24.** A composition according to claim 23, in which the composition is capable of increasing hair growth by a least 20%.

**25.** A process for the preparation of a composition according to any preceding claim which comprises to steps of:
(i) preparing fragments of hyaluronic acid, said fragments being characterised as polysaccharides containing from 7 to 50 monosaccharide units terminating either with a glucuronic acid unit and/or a N-acetyl glucosamine unit, or a unsaturated derivative of one or both of said terminal units; and
(ii) combining said fragments with a cosmetically acceptable vehicle.

**26.** A cosmetic method for converting vellus hair to growth as terminal hair which comprises the step of applying to the scalp in the region of vellus hair an effective amount of a composition according to any

of claims 1 to 24.

**27.** A cosmetic method for increasing the rate of terminal hair growth which comprises the step of applying to the scalp in the region of terminal hair an effective amount of a composition according to any of claims 1 to 24.

**Patentansprüche**

**1.** Zusammensetzung zur topischen Anwendung auf die Haut von Säugern, enthaltend:
(i) 0.01 bis 99 Gew.% Hyaluronsäure-Fragmente, die 7 bis 50 Monosaccharid-Einheiten enthalten, die entweder mit einer Glucuronsäure-Einheit und/oder einer N-Acetyl-glucosamin-Einheit, oder einem ungesättigten Derivat von einem oder-beiden dieser endständigen Einheiten abschließen; und
(ii) 1 bis 99.99 Gew.% eines kosmetisch verwendbaren Vehikels;
mit der Maßgabe, daß dann, wenn die Fragmente der Hyaluronsäure im wesentlichen aus Fragmenten bestehen, die aus mehr als 25 Monosaccharid-Einheiten zusammengesetzt sind, die Zusammensetzung außerdem ein Mittel enthält, das in der Folge einer topischen Anwendung der Zusammensetzung auf die Haut die Aktivität dieser Fragmente in bezug auf angiogenetische und/oder Haarwachstumswirkung verstärkt.

**2.** Zusammensetzung nach Anspruch 1, worin die Hyaluronsäure-Fragmente 7 bis 25 Monosaccharid-Einheiten enthalten.

**3.** Zusammensetzung nach Anspruch 2. worin die Zusammensetzung zusätzlich ein Mittel enthält, das in der Folge einer topischen Anwendung der Zusammensetzung auf die Haut die Aktivität dieser Fragmente in bezug auf angiogenetische und/oder Haarwachstumswirkung verstärkt.

**4.** Zusammensetzung nach Anspruch 1, 2 oder 3, enthaltend 0.1 bis 20 Gew.% Hyaluronsäure-Fragmente.

**5.** Zusammensetzung nach einem der vorhergehenden Ansprüche, worin das Vehikel 80 bis 99.9 Gew.% der Zusammensetzung bildet.

**6.** Zusammensetzung nach einem der vorhergehenden Ansprüche, worin das Mittel zur Aktivitätssteigerung der Fragmente der Hyaluronsäure ein Haarwachstums-Stimulant ist.

**7.** Zusammensetzung nach Anspruch 6, worin der Haarwachstums-Stimulant ausgewählt ist aus:
(i) $\alpha$-1,4-veresterte Disaccharide mit der Struktur (2);
(ii) veresterte Oligosaccharide, enthaltend mindestens eine veresterte Disaccharid-Einheit, bestehend aus einem Uronsäure-Rest mit der Struktur (3) und einem Hexosamin-Rest mit der Struktur (4);
(iii) Minoxidil und Derivate davon;
(iv) Direkte Proteoglykanase-Hemmer;
(v) Glykosaminoglykanase-Hemmer;
(vi) Glykosaminoglykan-Ketten-Zellaufnahme-Hemmer;
(vii) Glykosidasehemmer; und
(viii) Chemische Aktivatoren der Protein-Kinase-C-Enzyme;

**8.** Zusammensetzung nach Anspruch 7, worin der Haarwachstums-Stimulant Minoxidil ist.

**9.** Zusammensetzung nach Anspruch 7, worin der Glykosaminoglykanase-Hemmer ein Aldonolacton mit der Struktur (5) ist.

**10.** Zusammensetzung nach Anspruch 9, worin das Aldonolacton D-Glucaro-1,4-lacton ist.

**11.** Zusammensetzung nach Anspruch 7, worin der Glykosaminoglykanase-Hemmer ein Monosaccharid mit der Struktur (6) ist.

**12.** Zusammensetzung nach Anspruch 11, worin das Monosaccharid N-Acetylglucosamin ist.

**13.** Zusammensetzung nach Anspruch 7, worin der Glykosidasehemmer ein Lactam mit der Struktur (8) ist.

**14.** Zusammensetzung nach Anspruch 13, worin das Lactam D-Glucaro-1,5-lactam ist.

**15.** Zusammensetzung nach Anspruch 7, worin der chemische Aktivator der Protein-Kinase-C-Enzyme ein Diacylglycerin mit der Struktur (9) ist.

**16.** Zusammensetzung nach Anspruch 15, worin das Diacylglycerin 1,2-Dihexanoyl-sn-glycerin ist.

**17.** Zusammensetzung nach einem der Ansprüche 1 bis 5, worin das Mittel zu Steigerung der Aktivität der Fragmente der Hyaluronsäure ein Penetrationsverstärker ist.

**18.** Zusammensetzung nach Anspruch 17, worin der Penetrationsverstärker ausgewählt ist aus:
1-Dodecylazacycloheptan-2-on,
Dibutyl-sebacat,
2-Hydroxyoctansäure,
Estern der Pyroglutaminsäure mit der Struktur (10),
oberflächenaktiven Mittel.

**19.** Zusammensetzung nach einem der Ansprüche 1 bis 5, worin das Mittel zur Steigerung der Aktivität der Fragmente der Hyaluronsäure ein kationisches Polymer ist.

**20.** Zusammensetzung nach einem der Ansprüche 1 bis 5, worin das Mittel zur Steigerung der Aktivität der Fragmente der Hyaluronsäure eine iontophoretische Vorrichtung ist.

**21.** Zusammensetzung nach einem der vorhergehenden Ansprüche, die, falls sie topisch auf Rattenhaut aufgetragen wird, das für diesen Test gewählte Tiermodell, in der Lage ist, eine angiogenetische Wirkung zu induzieren, das heißt eine Steigerung der Entwicklung von Blutgefäßen in der Haut nach mindestens 5 Tagen, die mindestens 5 % größer ist als diejenige, die erreichbar ist, wenn man eine Kontrollzusammensetzung ohne die Fragmente der Hyaluronsäure verwendet.

**22.** Zusammensetzung nach Anspruch 21, worin die Zusammensetzung in der Lage ist, die Entwicklung der Blutgefäße um mindestens 10 % zu steigern.

**23.** Zusammensetzung nach einem der vorhergehenden Ansprüche, die, falls sie topisch auf Rattenhaut aufgetragen wird, das für diesen Test gewählte Tiermodell, in der Lage ist, eine Haarwachstums-Wirkung zu induzieren, das heißt eine Steigerung des Haarwuchses nach mindestens 14 Tagen, die mindestens 10 % größer ist als diejenige, die erreichbar ist, wenn man eine Kontrollzusammensetzung ohne die Fragmente der Hyaluronsäure verwendet.

**24.** Zusammensetzung nach Anspruch 23, worin die Zusammensetzung in der Lage ist, den Haarwuchs um mindestens 20 % zu steigern.

**25.** Verfahren zur Herstellung einer Zusammensetzung gemäß einem der vorhergehenden Ansprüche, das die Stufen umfaßt:
(i)) Herstellung von Fragmenten der Hyaluronsäure, wobei diese Fragmente als Polysaccharide mit 7 bis 50 Monosaccharid-Einheiten charakterisiert sind, die entweder mit einer Glucuronsäure-Einheit und/oder einer N-Acetyl-glucosamin-Einheit, oder einem ungesättigten Derivat von einem oder beiden dieser endständigen Einheiten abschließen; und
(ii) Kombinieren der genannten Fragmente mit einem kosmetisch verwendbaren Vehikel.

**26.** Kosmetisches Verfahren, damit Flaumhaar als Terminalhaar wächst, enthaltend die Stufe der Anwendung einer wirksamen Menge einer Zusammensetzung nach einem der Ansprüche 1 bis 24 auf die Kopfhaut im Bereich von Flaumhaar.

**27.** Kosmetisches Verfahren zur Steigerung der Geschwindigkeit des Wachstums von Terminalhaar, enthaltend die Stufe der Anwendung einer wirksame Menge einer Zusammensetzung nach einem der Ansprüche 1 bis 24 auf die Kopfhaut im Bereich von Terminalhaar.

**Revendications**

1. Composition pour l'application topique sur la peau de mammifère qui comprend :
(i) de 0,01 à 99 % en poids de fragments d'acide hyaluronique comprenant de 7 à 50 unités monosaccharides se terminant soit par une unité d'acide glucuronique et/ou une unité de N-acétylglucosamine, ou un dérivé insaturé de un ou de deux de ces unités terminales; et
(ii) de 1 à 99,99 % en poids d'un véhicule cosmétiquement acceptable;
à condition que quand les fragments d'acide hyaluroniques consistent essentiellement en fragments composés de plus de 25 unités monosaccharides, alors la composition comprend aussi un moyen pour augmenter l'activité desdits segments, en terme de réponse angiogénique et/ou de croissance des cheveux, après une application topique de la composition sur la peau.

2. Composition selon la revendication 1, dans laquelle les fragments d'acide hyaluronique comprennent entre 7 et 25 unités monosaccharides.

3. Composition selon la revendication 2 dans laquelle la composition comprend en plus un moyen pour augmenter l'activité desdits fragments après application topique sur la peau, en terme de réponse angiogénique et/ou de croissance des cheveux.

4. Composition selon la revendication 1, 2 ou 3 qui comprend entre 0,1 et 20 % en poids de fragments d'acide hyaluronique.

5. Composition selon l'une quelconque des revendications, dans laquelle le véhicule constitue de 80 à 99,9 % en poids de la composition.

6. Composition selon l'une quelconque des revendications précédentes dans laquelle le moyen destiné à accroître l'activité des fragments de l'acide hyaluronique est un stimulant de la croissance des cheveux.

7. Composition selon la revendication 6, dans laquelle on choisit le stimulant de croissance des cheveux parmi :
(i) les disaccharides $\alpha$-1,4 estérifiés ayant la structure (2);
(ii) les oligosaccharides comprenant au moins une unité disaccharide estérifiée qui consiste en un reste d'acide uronique qui a la structure (3) et en un reste hexosamine ayant la structure (4);
(iii) le minoxidil et ses dérivés;
(iv) des inhibiteurs directs de protéoglycanase;
(v) des inhibiteurs de glycosaminoglycanase;
(vi) des inhibiteurs d'absorption cellulaire à chaîne glycosaminoglycane;
(vii) des inhibiteurs de glycosidase; et
(viii) des activateurs chimiques d'enzymes protéine kinase C.

8. Composition selon la revendication 7, dans laquelle le stimulant de la croissance des cheveux est le minoxidil.

9. Composition selon la revendication 7, dans laquelle l'inhibiteur de glycosaminoglycanase est une aldonolactone ayant la structure (5).

10. Composition selon la revendication 9 dans laquelle l'aldonolactone est la D-glucaro-1,4-lactone.

11. Composition selon la revendication 7, dans laquelle l'inhibiteur de glycosaminoglycanase est un monosaccharide ayant la structure (6).

12. Composition selon la revendication 11, dans laquelle le monosaccharide est la N-acétylglucosamine.

13. Composition selon la revendication 7, dans laquelle l'inhibiteur de glycosidase est un lactame ayant la structure (8).

14. Composition selon la revendication 13, dans laquelle le lactame est le D-glucaro-1,5-lactame.

**15.** Composition selon la revendication 7, dans laquelle l'activateur chimique des enzymes protéine kinase C est un diacylglycérol ayant la structure (9).

**16.** Composition selon la revendication 15, dans laquelle le diacylglycérol est le 1,2-dihexanoyl-sn-glycérol.

**17.** Composition selon l'une quelconque des revendications 1 à 5, dans laquelle le moyen d'augmentation d'activité des fragments d'acide hyaluronique est un promoteur de pénétration.

**18.** Composition selon la revendication 17, dans laquelle le promoteur de pénétration est choisi parmi :
1-dodécylazacycloheptanone-2
sébaçate de dibutyle,
acide 2-hydroxyoctanoïque
esters de l'acide pyroglutamique ayant la structure (10)
agents tensioactifs.

**19.** Composition selon l'une quelconque des revendications 1 à 5, dans laquelle le moyen d'augmentation de l'activité des fragments d'acide hyaluronique est un polymère cationique.

**20.** Composition selon l'une quelconque des revendications 1 à 5, dans laquelle le moyen d'augmentation de l'activité des fragments de l'acide hyaluronique est un dispositif iontophorétique.

**21.** Composition selon l'une des revendications précédentes qu'on peut appliquer topiquement sur la peau du rat, l'animal modèle choisi pour ce test, qui est capable d'induire une réponse angiogénique, c'est-à-dire une augmentation dans le développement de vaisseaux sanguins dans la peau après au moins 5 jours d'au moins 5 % de plus que ce qu'on obtient avec une composition témoin qui a la même formulation sans les fragments d'acide hyaluronique.

**22.** Composition selon la revendication 21, dans laquelle la composition peut augmenter le développement du nombre des vaisseaux sanguins d'au moins 10 %.

**23.** Composition selon l'une quelconque des revendications précédentes qu'on applique topiquement sur la peau du rat, l'animal modèle choisi pour ce test, qui est capable d'induire une réponse de croissance de poils, c'est-à-dire une augmentation de croissance de poils après au moins 14 jours, d'au moins 10 % de plus que ce qu'on peut obtenir en utilisant une composition témoin qui a la même composition sans les fragments d'acide hyaluronique.

**24.** Composition selon la revendication 23, dans laquelle la composition peut augmenter la croissance des poils d'au moins 20 %.

**25.** Procédé de préparation d'une composition selon l'une quelconque des revendications précédentes qui comprend les étapes de :
(i) préparer les fragments d'acide hyaluronique, lesdits fragments étant caractérisés par des polysaccharides contenant de 7 à 50 unités monosaccharides se terminant soit par une unité d'acide glucuronique et/ou une unité de N-acétylglucosamine, ou un dérivé insaturé de un ou de ces deux unités terminales, et
(ii) combiner lesdits fragments avec un véhicule cosmétiquement acceptable.

**26.** Procédé cosmétique de conversion de duvet en cheveux par croissance qui comprend l'étape d'appliquer sur le cuir chevelu dans la région du duvet une quantité efficace d'une composition selon l'une quelconque des revendications 1 à 24.

**27.** Procédé cosmétique d'augmentation de la vitesse de croissance des cheveux qui comprend l'étape d'appliquer sur le cuir chevelu dans la région des cheveux une quantité efficace d'une composition selon l'une quelconque des revendications 1 à 24.